# EUROPEAN PATENT APPLICATION

(11) **EP 4 586 104 A1**
(43) Date of publication of application: **16.07.2025**
(21) Application number: 23925704.1
(22) Date of filing: 06.03.2023
(51) Int. Cl.: G06F 16/33, G06F 40/211, G06F 40/279, G06N 5/02

(54) **VARIATION EVIDENCE ITEM DETERMINATION METHOD AND APPARATUS, AND ELECTRONIC DEVICE**

(71) Applicant: BGI Genomics Co., Limited, Guangdong 518083 (CN)
(72) Inventor: ZENG, Quanlei, Shenzhen, Guangdong 518083 (CN); HUANG, Daoling, Shenzhen, Guangdong 518083 (CN); PANG, Chaoqun, Shenzhen, Guangdong 518083 (CN); LIU, Shuixia, Shenzhen, Guangdong 518083 (CN); LI, Hong, Shenzhen, Guangdong 518083 (CN); XIONG, Yun, Shenzhen, Guangdong 518083 (CN); YUAN, Yuying, Shenzhen, Guangdong 518083 (CN); LI, Ning, Shenzhen, Guangdong 518083 (CN)
(74) Representative: Kransell & Wennborg KB
(86) International application number: PCT/CN2023/079892
(87) International publication number: WO 2024/182979

(57) **Abstract**

The present application belongs to the field of variant interpretation. Disclosed are a variant evidence item determination method and apparatus, and an electronic device. The variant evidence item determination method comprises: extracting judgment content of at least one evidence item based on a target principle, to acquire at least one evidence item standard knowledge entry; representing the evidence item standard knowledge entry, so as to acquire an evidence item standard knowledge graph; identifying information recorded in a target document set, and matching the information with the evidence item standard knowledge graph, so as to acquire an evidence item knowledge graph; and based on the evidence item knowledge graph, determining a target evidence item corresponding to a target variant. The variant evidence item determination method of the present application significantly improves the determination efficiency, judgment accuracy and precision of an evidence item.

## Description

### FIELD

The present application belongs to the field of variant interpretation, and particularly, relates to a variant evidence item determination method and apparatus, and an electronic device.

### BACKGROUND

Variant interpretation is a medical evidence-based process in which detected sequence variants are classified in combination with clinical phenotypes, clinical knowledge bases, personalized medication guidelines and literature to determine their association with diseases, drugs, and individual characteristics and phenotypes. In the related art, the literature recording a target variant is retrieved from a large number of published documents mainly by means of manual retrieval and reading one by one, and then the specific content recording the target variant is further extracted from the retrieved literature to determine one or at least one evidence item of the target variant, and the type of the target variant is determined based on the evidence item. Such a process requires manual and repeated retrieval, reading, and judgment, which consumes a lot of manpower and time, requiring a relatively high cost and low sub-efficiency. In addition, the manual judgment is not only prone to omission of information, but also depends on personal professional knowledge reserves. Different interpreters may have different understandings of the same content, which will cause differences in results and is greatly affected by subjective factors, resulting in low final accuracy.

### SUMMARY

The present application aims to solve at least one of the technical problems existing in the prior art. To this end, the present application provides a variant evidence item determination method and apparatus, and an electronic device, for improving the efficiency of determining evidence items and the accuracy of judgment results.

In the first aspect, the present application provides a variant evidence item determination method. The method includes: extracting judgment content of at least one evidence item based on a target principle, to acquire at least one evidence item standard knowledge entry; representing the evidence item standard knowledge entry to acquire an evidence item standard knowledge graph; identifying information recorded in a target document set, and matching the information with the evidence item standard knowledge graph, to acquire an evidence item knowledge graph; and determining, based on the evidence item knowledge graph, a target evidence item corresponding to a target variant.

According to the variant evidence item determination method in the present application, the judgment content of at least one evidence item is extracted based on the target principle; the at least one evidence item standard knowledge entry acquired is represented to construct the evidence item standard knowledge graph; and the information recorded in the target document set is inferred based on the evidence item standard knowledge graph, to acquire the evidence item knowledge graph. In the subsequent application process, the required target evidence item corresponding to the target variant can be quickly and accurately acquired from massive literature, having a broad application perspective. In addition, the repeat manual search can be omitted, which saves manpower and time cost and improves the efficiency of determining evidence items. Besides, the influence of user's subjective judgment on final result can be effectively avoided, thereby improving the uniformity of the judgment result and having higher judgment accuracy and precision.

According to an embodiment of the present application, said representing the evidence item standard knowledge entry to acquire the evidence item standard knowledge graph includes: representing the evidence item standard knowledge entry with a triad as a basic unit, to acquire at least one evidence item standard knowledge representation entry; and combining the at least one evidence item standard knowledge representation entry, to acquire the evidence item standard knowledge graph.

According to an embodiment of the present application, the evidence item standard knowledge representation entry takes a variant entity as a head node and an evidence item as a tail node.

According to an embodiment of the present application, in the evidence item knowledge graph, respective nodes other than the tail node and edges are each represented by a specific name recorded in the target document set.

According to an embodiment of the present application, said identifying information recorded in a target document set, and matching the information with the evidence item standard knowledge graph, to acquire an evidence item knowledge graph includes: acquiring at least part of an entity and/or relationship comprised in an evidence item standard knowledge representation entry within a first target range; and in a case that the information recorded in the target document set matches the at least part of the entity and/or relationship, representing the evidence item standard knowledge representation entry to construct the evidence item knowledge graph.

According to an embodiment of the present application, said identifying information recorded in a target document set, and matching the information with the evidence item standard knowledge graph, to acquire an evidence item knowledge graph includes: determining, based on the target document set, a sentence containing an entity and/or relationship of a first triad in an evidence item standard knowledge representation entry as an evidence sentence; and in a case that other entities and/or relationships in the evidence item standard knowledge representation entry are acquired by querying a context of the evidence sentence, transforming, based on specific names corresponding to the other entities and/or relationships recorded in the target document set, the evidence item standard knowledge representation entry corresponding to the other entities and/or relationships, to generate the evidence item knowledge graph.

According to an embodiment of the present application, said identifying information recorded in a target document set, and matching the information with the evidence item standard knowledge graph, to acquire an evidence item knowledge graph includes: determining an evidence sentence based on a type of the evidence item standard knowledge representation entry; and generating the evidence item knowledge graph based on the evidence sentence and information recorded in the target document set.

According to an embodiment of the present application, said extracting, based on the target principle, the judgment content of the at least one evidence item, to acquire the at least one evidence item standard knowledge entry includes: extracting, based on the target principle, the judgment content of the at least one evidence item based on entities and relationships of the entities, to acquire the at least one evidence item standard knowledge entry. The entities include at least one of a variant entity, a disease entity, a patient entity, an index entity, a functional entity, or an evidence item.

In the second aspect, the present application provides a variant evidence item determination apparatus. The apparatus includes: a first processing module configured to extract judgment content of at least one evidence item based on a target principle, to acquire at least one evidence item standard knowledge entry; a second processing module configured to represent the evidence item standard knowledge entry to acquire an evidence item standard knowledge graph, wherein the number of items comprised in the evidence item standard knowledge graph matches the evidence item standard knowledge entry; a third processing module configured to: identify information recorded in a target document set, and match the information with the evidence item standard knowledge graph, to acquire an evidence item knowledge graph; and a fourth processing module configured to determine, based on the evidence item knowledge graph, a target evidence item corresponding to a target variant.

According to the variant evidence item determination apparatus in the present application, the judgment content of at least one evidence item is extracted based on the target principle; the at least one evidence item standard knowledge entry acquired is represented to construct the evidence item standard knowledge graph; and the information recorded in the target document set is inferred based on the evidence item standard knowledge graph, to acquire the evidence item knowledge graph. In the subsequent application process, the required target evidence item corresponding to the target variant can be quickly and accurately acquired from massive literature, having a broad application perspective. In addition, the repeat manual search can be omitted, which saves manpower and time cost and improves the efficiency of determining evidence items. Besides, the influence of user's subjective judgment on final result can be effectively avoided, thereby improving the uniformity of the judgment result and having higher judgment accuracy and precision.

In the third aspect, the present application provides an electronic device. The electronic device includes a memory, a processor, and a computer program stored on the memory and executable on the processor. The processor, when executing the computer program, implements the variant evidence item determination method according to the first aspect above.

In the fourth aspect, the present application provides a non-transitory computer-readable storage medium having a computer program stored thereon. The computer program, when executed by a processor, implements the variant evidence item determination method according to the first aspect above.

In the fifth aspect, the present application provides a chip including a processor and a communication interface. The communication interface is coupled to the processor. The processor is configured to execute a program or instructions for implementing the variant evidence item determination method according to the first aspect.

In the sixth aspect, the present application provides a computer program product including a computer program. The computer program, when executed by a processor, implements the variant evidence item determination method according to the first aspect above.

One or at least one technical solution in the embodiment of the present application as mentioned above has at least one of the following technical effects.

The judgment content of at least one evidence item is extracted based on the target principle; the at least one evidence item standard knowledge entry acquired is represented to construct the evidence item standard knowledge graph; and the information recorded in the target document set is inferred based on the evidence item standard knowledge graph, to acquire the evidence item knowledge graph. In the subsequent application process, the required target evidence item corresponding to the target variant can be quickly and accurately acquired from massive literature, having a broad application perspective. In addition, the repeat manual search can be omitted, which saves manpower and time cost and improves the efficiency of determining evidence items. Besides, the influence of user's subjective judgment on final result can be effectively avoided, thereby improving the uniformity of the judgment result and having higher judgment accuracy and precision.

Further, by setting the first target range, the matching range can be narrowed to improve the matching rate, thereby improving the efficiency of constructing the evidence item knowledge graph without affecting the precision of constructing the evidence item knowledge graph.

Furthermore, by transforming the evidence item standard knowledge representation entry corresponding to the sentence other than evidence sentence, and by supplementing the transformed result to the evidence item knowledge graph, a coverage range of the acquired evidence item knowledge graph can be effectively broadened, the correlation relationships of entities can be enriched, and functional attributes of the evidence item knowledge graph can be enhanced, thereby further improving the accuracy and precision of the final judgment results.

Still further, by setting different ways of confirming the evidence sentence based on different types of evidence item standard knowledge representation entries, a fast query of relevant content can be achieved, whiling reducing noise and improving the efficiency of constructing the evidence item knowledge graph.

Additional aspects and advantages of the present application will be set forth in part in the description which follows and, in part, will be obvious from the description, or may be learned by practice of the present application.

### BRIEF DESCRIPTION OF THE DRAWINGS

The foregoing and/or additional aspects and advantages of the present application will become apparent and readily understood from the following description of the embodiments, taken in conjunction with the accompanying drawings of which:
FIG. 1 is a schematic flowchart of a variant evidence item determination method according to an embodiment of the present application;
FIG. 2 is a schematic structural diagram of a variant evidence item determination apparatus according to an embodiment of the present application; and
FIG. 3 is a schematic structural diagram of an electronic device according to an embodiment of the present application.

### DETAILED DESCRIPTION

Embodiments of the present application are described clearly hereinafter with reference to the accompanying drawings, in which some, but not all, of the embodiments of the present application are illustrated. Based on the embodiments in the present application, all other embodiments acquired by a person skilled in the art shall fall within the scope of protection of the present application.

The terms "first", "second", etc. in the description and in the claims of the present application are used solely for distinguishing similar objects and do not imply any specific order or precedence. It can be understood that the data used are interchangeable under appropriate circumstances such that the embodiments of the present application can be practiced in sequences other than those illustrated or described herein, and that the terms "first", "second", etc. are generally intended to refer to one type of object, and not to a limitation on the number of objects, e.g., a first object may be one or more. Furthermore, in the description and the claims the term "and/or" indicates at least one of the connected objects, and the symbol "/" generally indicates that the associated object is an "or" relationship.

A variant evidence item determination method, A variant evidence item determination apparatus, an electronic device, and a readable storage medium provided by the embodiments of the present application will be described in detail through specific embodiments and application scenarios thereof with reference to the accompanying drawings.

The variant evidence item determination method can be applied to a terminal, and can be specifically executed by hardware or software in the terminal.

The terminal includes, but is not limited to, a portable communication device such as a mobile phone or tablet. It should also be understood that, in some embodiments, the terminal may not be a portable communication device, but a desktop computer.

It should be understood that the terminal may include one or more other physical user interface devices such as touch sensitive surfaces, physical keyboards, mice, and joysticks.

For the variant evidence item determination method provided in an embodiment of the present application, an execution subject of the variant evidence item determination method may be an electronic device, or a functional module or entity in an electronic device capable of implementing the variant evidence item determination method. The electronic device mentioned in the embodiments of the present application includes, but is not limited to, a mobile phone, a tablet computer, a computer, a camera and an intelligent device, etc. For illustrative purposes, the variant evidence item determination method provided in the embodiment of the present application is described below using an electronic device as the execution subject.

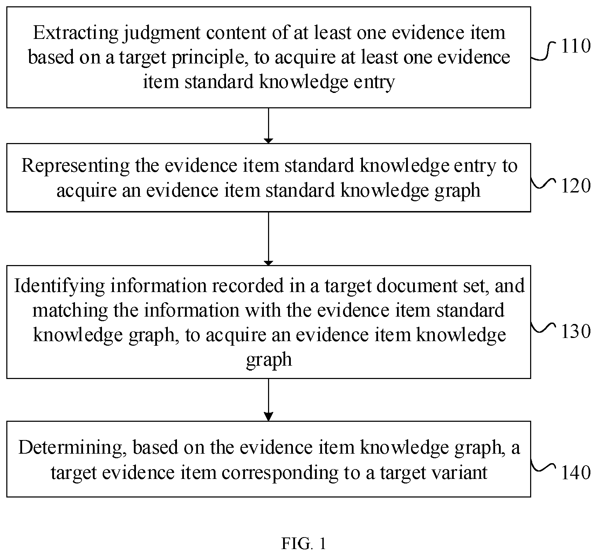

As illustrated in FIG. 1, the variant evidence item determination method includes Step 110, Step 120, Step 130, and Step 140.

Step 110: Extract the judgment content of at least one evidence item based on a target principle to acquire at least one evidence item standard knowledge entry.

In this step, the target principle may include: principles proposed by authoritative industry organizations or institutions or widely recognized, as well as user-defined principles.

The principles proposed by authoritative industry organizations or institutions or widely recognized may include, but are not limited to: industry standards, expert consensuses, guidelines, and expert recommendations, which are not limited herein.

Industry standards refer to relevant standards within gene detection industry.

For example, the industry standard may be an authoritative genetic variant classification criteria and guidelines, such as, the Standards and guidelines for the interpretation of sequence variants (2015) published by the American College of Medical Genetics and Genomics (ACMG) (Richards S, Aziz N, Bale S, et al. Standards and guidelines for the interpretation of sequence variants: a joint consensus recommendation of the American College of Medical Genetics and Genomics and the Association for Molecular Pathology[J]. Genetics in medicine, 2015, 17(5): 405-423.). This guideline specifies 28 criteria (termed "evidence items") for variant classification and establishes standardized rules for combining these criteria. Based on these rules, the sequence variants can be classified into pathogenic, likely pathogenic, benign, likely benign, and variants of uncertain significance.

Expert consensus refers to information such as the consensus reached by relevant experts in the industry.

Evidence items are criteria used to determine the classification of target variant.

In some embodiments, step 110 may include: based on the target principle, the judgment content of the at least one evidence item is extracted based on entities and relationships of the entities, to acquire the at least one evidence item standard knowledge entry. Entities include one or more of the following: a variant entity, a disease entity, a patient entity, an index entity, a functional entity, or an evidence item.

In this embodiment, the evidence item standard knowledge entry consists of at least two entities and relationships between the entities.

Entities include one or more of the following: a variant entity, a disease entity, a patient entity, an index entity, a functional entity, or an evidence item.

The variant entity is a locus that differs from the reference genome.

The naming convention can adhere to the naming rules in the industry.

For example, for a specific variant site, the variant entity may be named as MSH2 c.2503A>C and MSH2 p.Asn835His, where: MSH2 is the gene name; and c.2503A>C and p.Asn835His indicate the alterations induced by the variant site in terms of cDNA level and protein level, respectively.

A disease entity is a medically-identified disease.

Standard names of disease entities can be acquired from public databases such as: https://www.orpha.net/consor/cgi-bin/index.php, as well as https://rarediseases.info.nih.gov/.

A patient entity is an individual or group of individuals clinically diagnosed with a disease.

The patient entity may include a patient, a proband, and a case.

The index entity is an evaluation index characterized by numerical value or numerical value interval, for example, odds ratio (OR), confidence interval (CI), etc.

Functional entity is the target of functional index in experimental study.

For example, if the activity of a protein is decreased, the corresponding functional entity is the activity of the protein; and if the transcript of a gene is truncated, the corresponding functional entity is the transcript.

Evidence items are criteria used to determine the type of target variant.

In some embodiments, descriptions and qualifying requirements for each entity, as attributes of the entity, may also be written into the evidence item standard knowledge representation entry.

In this embodiment, the attributes corresponding to the disease entity may include the association between disease and gene, the association between disease and phenotype, the penetrance of the disease, and its inheritance pattern, etc.

The attribute corresponding to the index entity can be a predefined range of values.

In practical implementation, a configuration library can be set to retrieve attributes corresponding to the required entities, and these attributes are attribute contents that need to be acquired by querying or integrating public information.

For example, a configuration library can be set up for disease entities, and the contents recorded in the configuration library include, but are not limited to, the association between gene and disease, the association between disease and phenotype, and the disease penetrance and inheritance pattern, etc.

The configuration library may be derived from one or at least one public database, e.g., from https://www.omim.org/.

According to the variant evidence item determination method provided in the embodiments of the present application, based on the target principle, the judgment content of at least one evidence item is extracted based on entities and relationships between entities, to acquire at least one evidence item standard knowledge entry, and the target principle is subjected to a structuring representation to generate the evidence item standard knowledge entry to be used for the construction of a subsequent knowledge graph, which is conducive to the improvement of the accuracy and precision of the knowledge graph.

Step 120: the evidence item standard knowledge entry is represented, to acquire an evidence item standard knowledge graph.

In this step, the evidence item standard knowledge graph is used to provide an evidence item judgment logic path for variant interpretation, thereby achieving rapid reasoning and response of knowledge.

It can be appreciated that: the at least one evidence item standard knowledge entry can be acquired in Step 110, and through the graph representation of the relevant evidence item standard knowledge entry in the at least one evidence item standard knowledge entry, an evidence item standard knowledge graph can be acquired.

The number of items included in the evidence item standard knowledge graph matches the evidence item standard knowledge entry.

In some embodiments, Step 120 may include: the evidence item standard knowledge entry is represented with a triad as a basic unit, to acquire at least one evidence item standard knowledge representation entry; and the at least one evidence item standard knowledge representation entry is combined, to acquire the evidence item standard knowledge graph.

In this embodiment, the triad is a knowledge graph triad.

The triad includes nodes corresponding to two adjacent entities and relationships between the two adjacent entities.

The evidence item standard knowledge representation entry is a representation acquired by connecting relevant partial evidence item standard knowledge entries in a triad as a basic unit.

As an example, in the Standards and guidelines for the interpretation of sequence variants, the specific content about the evidence item standard knowledge representation entry is as follows: the first evidence item standard knowledge representation entry may include supporting evidence of pathogenicity (PP1, PP5), strong evidence of benign impact (BS4), and supporting evidence of benign impact (BP5, BP6); the second evidence item standard knowledge representation entry may include strong evidence of pathogenicity (PS3, PS4), supporting evidence of pathogenicity (PP4), and strong evidence of benign impact (BS3); and the third evidence item standard knowledge representation entry may include supporting evidence of pathogenicity (PS2), moderate evidence of pathogenicity (PM3, PM6), and supporting evidence of benign impact (BP2).

According to the characteristics of the respective evidence items, a targeted design of the representation entries is performed to provide a reasonable and efficient path for the reasoning of evidence items.

The evidence item standard knowledge representation entry may include one or more triads.

In a case that the evidence item standard knowledge representation entry includes a plurality of triads, the plurality of triads are connected according to a certain structure.

Different evidence item standard knowledge representation entries may be different in structure.

It will be appreciated that different evidence item standard knowledge representation entries may be acquired based on different evidence item standard knowledge entries.

Evidence item standard knowledge graph consists of a plurality of evidence item standard knowledge representation entries.

After acquiring at least one different evidence item standard knowledge representation entry, the at least one different evidence item standard knowledge representation entry is associated and combined to acquire the evidence item standard knowledge graph.

In practical implementation, the respective evidence item standard knowledge entries may be processed one by one or in batch.

In some embodiments, the evidence item standard knowledge representation entry takes a variant entity as a head node and an evidence item as a tail node.

In this embodiment, the variant entity is a site that differs from the reference genome.

Evidence items are criteria used to determine the type of variant.

The relationship of the respective nodes is the relationship between the entities corresponding to two adjacent nodes.

In some embodiments, the evidence item standard knowledge entry may also include at least one intermediate node.

In this embodiment, the intermediate node corresponds to an intermediate entity, and the intermediate entity may include a variant entity, a disease entity, a patient entity, an index entity and a functional entity, etc.

For example, the first evidence item standard knowledge representation entry may include a head node of the variant entity representation, a tail node of the evidence item representation, and an edge of the representation of the relationship between the variant entity and the evidence item.

For example, the second evidence item standard knowledge representation entry may include a head node of the variant entity representation, an intermediate node of one or more intermediate entity representations, a tail node of an evidence item representation, and an edge of a relationship representation of each node, and each intermediate node has only one parent node and/or child node.

For example, the third evidence item standard knowledge representation entry may include a head node of the variant entity representation, an intermediate node of one or more intermediate entity representations, a tail node of an evidence item representation, and an edge of a relationship representation of each node, and at least one intermediate node has two or more parent nodes and/or child nodes.

The parent nodes refer to adjacent nodes in the direction of the path head node, and the child nodes refer to adjacent nodes in the direction of the path tail node.

It will be appreciated that: different evidence item standard knowledge representation entries may be different in structure, and the differences may include nodes and the number of nodes, edges and the number of edges, and the number of triads, etc.

In practical implementation, it can be determined according to the sequence in the direction from the head node to the tail node that the respective intermediate nodes are a first intermediate node and a second intermediate node, etc., and correspond to a first intermediate entity and a second intermediate entity.

For example, an evidence item standard knowledge representation entry item A includes a head node and a tail node; an evidence item standard knowledge representation entry item **B** includes a head node, a first intermediate node, and a tail node; and an evidence item standard knowledge representation entry item **C** includes a head node, a first intermediate node, a second intermediate node, and a tail node.

In the direction from the head node to the tail node, the relationship between the variant entity and the corresponding entity of the next node is taken as a first relationship, represented as a first edge, and so on.

For example, for the certain evidence item standard knowledge representation entry A only including a head node and a tail node, a first relationship is a relationship between a variant entity and the evidence item, corresponding to a first edge in the evidence item standard knowledge representation entry.

For the evidence item standard knowledge representation entry **B** containing a head node, a first intermediate node, and a tail node, a first relationship is a relationship between a variant entity and the first intermediate entity, and a second relationship is a relationship between the first intermediate entity and the evidence item, sequentially corresponding to a first edge and a second edge in the evidence item standard knowledge representation entry.

For the evidence item standard knowledge entry **C** containing a head node, a first intermediate node, a second intermediate node, and a tail node, a first relationship is a relationship between a variant entity and the first intermediate entity, a second relationship is a relationship between the first intermediate entity and the second intermediate entity, and a third relationship is a relationship between the second intermediate entity and the evidence item, sequentially corresponding to a first edge, a second edge and a third edge in the evidence item standard knowledge representation entry in sequence.

In the direction from the head node to the tail node, a first triad and a second triad are successively formed according to the combination of the respective nodes and the relationship.

For example, for a certain evidence item standard knowledge representation entry A only including a head node and a tail node, a first triad is only contained, i.e., a head node, a tail node and a first edge.

For example, for the evidence item standard knowledge representation entry B including a head node, a first intermediate node, and a tail node, the included first triad includes a head node, a first intermediate node, and a first edge, and the included second triad includes a first intermediate node, a tail node, and a second edge.

If there are differences in the information recorded in different evidence item standard knowledge entries, the structure of the finally acquired represented evidence item standard knowledge representation entries may also be different.

For example, in a case that an evidence item standard knowledge entry **X** consists of a variant entity, an evidence item, and a relationship between the variant entity and the evidence item, the variant entity is taken as a head node, the evidence item is taken as a tail node, and the relationship between the variant entity and the evidence item is written in the evidence item standard knowledge graph as an edge.

For another example, in a case that an evidence item standard knowledge entry **Y** consists of a variant entity, a disease entity, an evidence item, a relationship between the variant entity and the disease entity, and the relationship between the disease entity and the evidence item, the variant entity is taken as a head node, the disease entity is taken as an intermediate node, the evidence item is taken as a tail node, and the relationship between the variant entity and the disease entity as well as the relationship between the disease entity and the evidence item are written in the evidence item standard knowledge graph as edges.

As an example, the supporting evidence of pathogenicity PP1 is defined in the Standards and guidelines for the interpretation of sequence variants as "cosegregation with disease in multiple affected family members in a gene definitively known to cause the disease".

Based on the definition, the PP1 evidence item standard knowledge entry can be extracted. The entities contained therein are variant entities and PP1 evidence items; and the relationship contained therein is a relationship between the variant entities and the PP1 evidence items. The PP1 evidence item standard knowledge entry is represented in the form of a triad. By taking the variant entity as a head node, the PP1 evidence item as a tail node, and the relationship between the variant entity and the PP1 evidence item as an edge, the PP1 evidence item standard knowledge representation entry can be acquired, which belongs to the first evidence item standard knowledge representation entry. The first evidence item standard knowledge representation entry corresponding to PP1 is written into the evidence item standard knowledge graph.

As an example, the supporting evidence of pathogenicity PS4 is defined in the Standards and guidelines for the interpretation of sequence variants as "the prevalence of the variant in affected individuals is significantly increased compared with the prevalence in controls".

Based on the definition, the PS4 evidence item standard knowledge representation entry is extracted. The entities contained therein include a variant entity, an index entity, and a PS4 evidence item; and the relationship contained therein includes a relationship between the variant entity and the index entity and a relationship between the index entity and the PS4 evidence item. The PS4 evidence item standard knowledge representation entry is represented in the form of triad. By taking the variant entity as a head node, the index entity as a first intermediate node, the PS4 evidence item as a tail node, the relationship between the variant entity and the index entity as a first edge, and the relationship between the index entity and the PS4 evidence item as a second edge, the PS4 evidence item standard knowledge representation entry can be acquired.

The index entity may be OR, CI, or a combination thereof. Meanwhile, a specific threshold value is set for OR and CI, for example, OR is greater than 5, and CI numerical value interval does not contain 1. That is, only when the index entity exists and satisfies the threshold requirement, the PS4 evidence item can be inferred based on the PS4 evidence item standard knowledge representation entry.

The evidence item standard knowledge representation entry consists of two triads, including one intermediate node, and the intermediate node has only one parent node and one child node, belonging to a second evidence item standard knowledge representation entry. The second evidence item standard knowledge representation entry corresponding to PS4 is written into the evidence item standard value knowledge graph.

As an example, the strong evidence of pathogenicity PS2 is defined in the Standards and guidelines for the interpretation of sequence variants as "de novo (both maternity and paternity confirmed) in a patient with the disease and no family history". Based on the definition, the PS2 evidence item standard knowledge entry is extracted. The entry contained therein includes a variant entity, a patient entity, and a PS2 evidence item; and the relationship contained therein includes a relationship between the variant entity and the patient entity and a relationship between the patient entity and the PS2 evidence item. The PS2 evidence item standard knowledge representation entry is represented in the form of triad. By taking the variant entity as a head node, the patient entity as a first intermediate node, the PS2 evidence item as a tail node, the relationship between the variant entity and the patient entity as a first edge, and the relationship between the patient entity and the PS2 evidence item as a second edge, the PS2 evidence item standard knowledge representation entry can be acquired.

According to the Standards and guidelines for the interpretation of sequence variants, for a new variant, the evidence item PS2 is met when a parent-child relationship is confirmed; evidence item PM6 (Assumed de novo, but without confirmation of paternity and maternity) is met a when parent-child relationship cannot be confirmed.

The same method is used to extract and represent the evidence item standard knowledge entry of PM6, and integrate with the PS2 evidence item standard knowledge representation entry. In the PS2 and PM6 evidence item standard knowledge representation entries, there are two child nodes after the patient entity, i.e., taking the PS2 evidence item as the tail node, taking the PM6 evidence item as the tail node, belonging to the third evidence item standard knowledge representation entry.

The third evidence item standard knowledge representation entry corresponding to PS2 and PM6 is written into the evidence item standard value knowledge graph.

According to the variant evidence item determination method provided in an embodiment of the present application, a variant entity is taken as a head node, an evidence item is taken a tail node, and a relationship between each node is taken as an edge to connect each node in sequence to generate an evidence item standard knowledge entry with high accuracy and precision.

Step 130: Information recorded in a target document set is identified and matched with the evidence item standard knowledge graph, to acquire an evidence item knowledge graph.

In this step, the target document set may be any relevant one or more document sets.

The types of documents may include, but are not limited to, contribution of monographs, newspaper articles, journal articles, dissertations, research reports, standards, patents, monographs, and paper collections, which is not limited herein.

The information recorded in the target document set may be any one or more nodes or one or more relationships (including variant entities, disease entities, patient entities, index entities and functional entities, etc.) other than evidence in the evidence item knowledge graph.

In practical implementation, a one or more specific documents can be acquired through manual selection, for example, arbitrary one or more documents, one or more documents that record target variant and are manually retrieved, one or more documents that record relevant diseases and are manually retrieved. When the target document set includes multiple documents, the target document set can also be a combination of the above. The content of the target document set is not limited in the present application.

In some embodiments, when the target document set includes multiple documents, all published documents may also be collected, with no limitation on collection channels or methods, such as may be acquired from a copyright owner of the document.

It can be understood that, for different target document sets, the acquired evidence item knowledge graphs are different.

In practical implementation, the number of items included in the evidence item standard knowledge graph matches with the evidence item standard knowledge entry; and the number of evidence item standard knowledge entry shall be greater than or equal to the items included in the evidence item knowledge graph finally acquired.

In practical implementation, the nodes and relationships involved in the evidence item standard knowledge graph can be matched with the content in the target document set, and in the case of successful matching, the nodes and relationships in the evidence item standard knowledge graph can be rewritten with the expression in the target document set, to obtain the evidence item knowledge graph.

For example, for one evidence item standard knowledge entry, the identification and extraction can be performed based on the entities and edges recorded in the evidence item standard knowledge entry. In a case that all the entities and edges can be matched, the entities and edges are represented using the information in the matched target document set, to acquire the evidence item knowledge graph.

It should be noted that the evidence item knowledge graph constructed in Step 130 varies with the changes of the actual recorded information in the target document set. The evidence item knowledge graph may include one or more variants (variants of the head node) to objectively present all the identifiable and matchable contents recorded in the target document set.

Different evidence item knowledge graphs can be constructed for different information recorded in the target document set.

In practical implementation, a plurality of evidence item knowledge graphs may be constructed based on Step 110 to Step 130.

The plurality of constructed evidence item knowledge graphs can be stored in the local or cloud database, to be invoked when needed.

In some embodiments, in the evidence item knowledge graph, respective nodes other than the tail node and edges are each represented by a specific name recorded in the target document set.

In this embodiment, the evidence item knowledge graph consists of evidence item representation entries transformed from evidence item standard knowledge representation entries.

It can be understood that, in a case that the recorded information in all the documents in the target document set does not match the information of any evidence item standard knowledge representation entry, it is considered that the requirements of all the evidence items are not met, and the evidence item representation entry contained in the evidence item knowledge graph is 0.

It should be noted that for the same target variant, there may be differences in the evidence item knowledge graph under different circumstances, and the differences are closely related to the target document set.

For example, when the information recorded in the target document set does not match any evidence item standard knowledge entry, the evidence item representation entry in the evidence item knowledge graph is 0.

For another example, when the target document set is updated (e.g., newly added, changed) and the information recorded therein matches a certain evidence item standard knowledge entry, i.e., meeting the requirements of the evidence item, the corresponding evidence item standard knowledge representation entry may be transformed into an evidence item representation entry and written into an evidence item knowledge graph.

When constructing an evidence item knowledge graph for at least one different piece of information, the evidence item knowledge graph corresponding to different pieces of information may be different.

For example, the information about information A recorded in the target document set coincides with the evidence item standard knowledge entry X, i.e., meeting the requirements of the evidence item, the corresponding evidence item standard knowledge representation entry X can be transformed into an evidence item representation entry and written into an evidence item knowledge graph.

For another example, if the information about other information B recorded in the target document set does not match the same evidence item standard knowledge entry X, i.e., failing to meet the requirements of the evidence item, the evidence item representation entry corresponding to the evidence item standard knowledge entry X is not included in the evidence item knowledge graph.

In some embodiments, when there is at least one piece of information, for the specific construction process, the at least one piece of information may be operated one by one or in batch. A specific case of performing a batch operation on the at least one piece of information may be to generate an evidence item knowledge graph of all the variants recorded in the target document set.

It can be understood that in the document set, specific names of entities are described in various forms, and that there are a number of aliases and irregular descriptions.

The description of specific names of the entities has been illustrated in the above embodiments and will not be repeated here.

Step 140: based on the evidence item knowledge graph, a target evidence item corresponding to a target variant is determined.

In this step, the target variant is a variant to be interpreted. For example, the target variant can be a variant of the evidence item of interest or to be determined, etc., which is not limited herein.

The target evidence items refer to the evidence items determined based on the constructed evidence item knowledge graph, including: specific evidence items included in the constructed evidence item knowledge graph, and null values.

The specific evidence items are the evidence items meeting the requirements in the constructed evidence item knowledge graph. The null value means that there is no qualified evidence.

According to the variant evidence item determination method in the present application, the judgment content of at least one evidence item is extracted based on the target principle, the acquired at least one evidence item standard knowledge entry is represented to construct an evidence item standard knowledge graph, and the information recorded in the target document set is inferred based on the evidence item standard knowledge graph, to acquire the evidence item knowledge graph. In the subsequent application process, the required target evidence item corresponding to a target variant can be acquired quickly and accurately from a large number of documents, and the application scenario is wide; there is no need to repeat the search manually, which saves manpower and time cost and improves the efficiency of determining evidence items. Besides, it effectively avoids the influence of user's subjective judgment on final result, improves the uniformity of the judgment result, and has higher judgment accuracy and precision.

In some embodiments, the method may further include: updating a target document set.

In this embodiment, an update mode may be set, such as a real-time update or a periodic update, which is not limited herein.

The evidence item standard knowledge graph includes at least one evidence item standard knowledge representation entry.

The evidence item knowledge graph includes at least one evidence item representation entry.

It should be noted that the number of evidence item representation entries included in the evidence item knowledge graph may be any non-negative integer, for example, 0, 1 or more, which is not limited herein.

In a case that the information corresponding to the target variant recorded in the target document set matches the evidence item standard knowledge representation entry in the evidence item standard knowledge graph, the evidence item standard knowledge representation entry is represented based on the specific name recorded in the target document set, to construct the evidence item knowledge graph.

In other embodiments, in a case that the recorded information of all the documents in the target document set does not match the information of any evidence item standard knowledge representation entry, it is considered that the requirements for all the evidence items are not met.

In some embodiments, Step 130 may further include: acquiring at least part of the entity and/or relationship included in an evidence item standard knowledge representation entry within a first target range; and in a case that the information recorded in the target document set matches the at least part of the entity and/or relationship, representing the evidence item standard knowledge representation entry to construct the evidence item knowledge graph.

In this embodiment, the first target range is a preset range, which may be defined by a user, such as within 7 sentences defined as the same paragraph.

At least part of the entity and/or relationship may represent all the entities and/or relationships that are included in the evidence item standard knowledge entry within the first target range and do not include a tail node.

For example, all entities and relationships of evidence item standard knowledge representation entries are queried within a preset range; if all the entities and relationships are included, the evidence item standard knowledge representation entries are transformed into the evidence item standard knowledge representation entries and written into the evidence item knowledge graph.

According to the variant evidence item determination method provided in the embodiments of the present application, by setting the first target range, the matching range can be narrowed to improve the matching rate, thereby improving the efficiency of constructing an evidence item knowledge graph without affecting the precision of constructing the evidence item knowledge graph.

In some embodiments, step 130 may include: determining, based on the target document set, a sentence containing an entity and/or relationship of a first triad in an evidence item standard knowledge representation entry as an evidence sentence; and in a case that other entities and/or relationships in the evidence item standard knowledge representation entry are acquired by querying a context of the evidence sentence, transforming, based on specific names corresponding to the other entities and/or relationships recorded in the target document set, the evidence item standard knowledge representation entry corresponding to the other entities and/or relationships, to generate the evidence item knowledge graph.

In this embodiment, the first triad is a triad where the head node is located.

The entity and/or relationship of the first triad may include numerous manners such as entities only, relationships only, and combinations of relationships and entities.

The entity and/or relationship of the first triad may be: the variant entity; either a first intermediate entity or a combination of the variant entity and the first intermediate entity; a first relationship; a combination of the first relationship and the variant entity; and a combination of the first relationship and the first intermediate entity.

For example, the entity may be a variant entity and/or a first intermediate entity, and the relationship is a first relationship.

Other entities and/or relationships are relationships and/or entities (other than evidence items) that are not used in determining evidence sentences.

Other entities and/or relationships may include: unused relationships and/or entities in the first triad in the evidence item standard knowledge representation entry; or unused relationships and/or entities in other triads in the evidence item standard knowledge representation entry.

For example, the evidence item standard knowledge representation entry A includes a first triad, and in a case that an evidence sentence is determined only using the variant entity of the first triad, it is still required to query in the context of the evidence sentence whether there is matching content for the first relationship not used in the first triad.

As another example, the evidence item standard knowledge representation entry item B includes a first triad and a second triad, and in a case that only the first relationship of the first triad is used to determine the evidence sentence, it is required to query whether there is matching content in the context of the evidence sentence among the variant entity, the first intermediate entity and the second relationship.

If the matching content is acquired by querying, the evidence item standard knowledge representation entries corresponding to the variant entity, the first intermediate entity and the second relationship that have the matching content are transformed to obtain the evidence item knowledge entries corresponding to the variant entity, the first intermediate entity and the second relationship, and these evidence item knowledge entries are supplemented into the evidence item knowledge graph.

According to the variant evidence item determination method provided in the embodiments of the present application, by transforming the evidence item standard knowledge representation entry corresponding to the sentence other than evidence sentence, and by supplementing the transformed results to the evidence item knowledge graph, a coverage range of the acquired evidence item knowledge graph can be effectively broadened, the correlation relationships of entities can be enriched, and functional attributes of the evidence item knowledge graph can be enhanced, thereby further improving the accuracy and precision of the final judgment results.

In some embodiments, said acquiring the other entities and/or relationships in the evidence item standard knowledge representation entries by querying the context of the evidence sentence may include: acquiring other entities and/or relationships in the evidence item standard knowledge representation entry by querying the context of the evidence sentence within the second target range.

In this embodiment, the second target range may be a preset range. For example, the second target range may be set as an evidence sentence and 3 or 5 sentences before and after the same paragraph of the evidence sentence, etc., which is not limited herein.

For another example, in practical implementation, a query can also be performed in the first sentence, the second sentence and the third sentence of the context of the same paragraph in sequence, and the query can be stopped when the relevant content is matched.

In some embodiments, document information (e.g., document unique identifier PMID, document title, document publication date, etc.), evidence sentence, and target range sentence that records the variant entity (head node) may also be written into the evidence item knowledge graph as attributes of the variant entity.

Optionally, the variant entities and their attributes included in the document set are summarized to generate a corresponding knowledge base, which is convenient to call and can be used for query and verification.

In practical implementation, the sentence of the entity and/or relationship corresponding to the triad where the head node is located can be determined as an evidence sentence, and other entities and/or relationships in the evidence item standard knowledge representation entry are queried in the context for the evidence sentence. If other entities and/or relationships are contained, the evidence item standard knowledge representation entry is transformed into an evidence item representation entry and written into the evidence item knowledge graph.

It should be noted that said determining that the sentence containing the entity and/or relationship of the first triad in the evidence item standard knowledge representation entry is an evidence sentence and said querying other entities and/or relationships in the evidence item standard knowledge representation entry in the context of the evidence sentence both involve an operation on at least one of the entity and relationship. In practical implementation, the entities and relationships need to be identified and extracted respectively.

In the present application, the identification method of an entity is not limited.

The identification may be performed, for example, by retrieving a specific lexicon or may also be performed by pre-training the model.

First, entity identification is performed using a specific lexicon

When using a specific lexicon for entity identification, the source of the lexicon may include, but is not limited to, document, public databases, etc.

For example, for patient entities, common descriptions can be collected from the document to build a lexicon.

The description of the patient may include patient, case, individual, object, etc.

In some embodiments, the lexicon may also be enriched according to part of speech transformation rules.

In some embodiments, the lexicon may also be extended with synonyms, paronyms, and antonyms.

Second, the identification is performed by pre-training the model

When a pre-training model is used for entity identification, there is no limitation on the type of the pre-training model, such as a neural network model.

Taking a variant entity as an example, a pre-training model capable of identifying the variant entity can be obtained by training with documents having variant labeling information, and then the pre-training model is used to identify the variant entity in other documents.

In some embodiments, after an entity and/or relationship is identified, the identified specific names may be further aligned.

In this embodiment, the identified specific names may be aligned, to align the identified entity names to corresponding entity standard terms.

Taking the variant entity as an example, the standard terminology of the variant entity can be named in a manner following a rule recorded in Human Genome Variant Society, HGVS, https://www.hgvs.org/. The method of entity alignment is not limited, for example, by regular expressions.

In the present application, the method of relationship identification is not limited.

The identification may be performed, for example, by building a lexicon for relationship keywords.

Specifically, sources of the relationship keywords may include, but are not limited to, document and public databases.

Taking the PS3 evidence item "Well-established in vitro or in vivo functional studies supportive of a damaging effect on the gene or gene product" as an example, the keyword "damaging" describing the relationship can be collected from the scientific literature to establish a lexicon of common descriptions, which may include impaired, aberrant, defective, disrupt, etc.

In some embodiments, the lexicon may also be enriched according to part of speech transformation rules.

In some embodiments, the lexicon may also be extended with synonyms, paronyms, and antonyms.

In some embodiments, step 130 may further include: determining an evidence sentence based on a type of the evidence item standard knowledge representation entry; and generating the evidence item knowledge graph based on the evidence sentence and information recorded in the target document set.

In this embodiment, based on the type, the at least one evidence item standard knowledge representation entry may be divided into a first evidence item standard knowledge representation entry, a second evidence item standard knowledge representation entry, and a third evidence item standard knowledge representation entry, etc.

The entity and/or relationship of the first triad include at least one of a first relationship, a variant entity, or a first intermediate entity.

For the first evidence item standard knowledge representation entry, the first relationship can be used to determine the evidence sentence.

For the second evidence item standard knowledge representation entry, at least a first intermediate entity can be used to determine an evidence sentence, and a first relationship or a variant entity located at a head node can also be used to determine the evidence sentence.

For the third evidence item standard knowledge representation entry, the first relationship may be used to determine the evidence sentence. According to the variant evidence item determination method provided in the embodiments of the present application, by providing different ways of confirming evidence sentences according to different types of evidence item standard knowledge representation entries, a fast query of relevant content can be achieved, whiling reducing noise and improving the efficiency of constructing the evidence item knowledge graph.

The construction process of the evidence item knowledge graph corresponding to relevant information is described below through specific examples.

Evidence item standard knowledge representation entries for evidence item 1 include: a variant entity (head node), an evidence item 1 (tail node), and a variant entity-evidence item relationship 1 (first edge).

Relevant information in document 1 in the target document set is identified using a pre-training model to determine a sentence containing relevant information as an evidence sentence, and the variant entity-evidence item relationship 1 is identified through a relationship keyword lexicon of the relationship 1 within the scope ranging from 1 sentence before to 1 sentence after the evidence sentence, and a matching relationship keyword 1 is queried.

According to the evidence item standard knowledge representation entry of the evidence item 1, it can be derived that what is recorded in document 1 meets the requirements of the evidence item 1, and then the evidence item standard knowledge representation entry of the evidence item 1 is transformed to form an evidence item representation entry, in which the variant entity is taken as a head node, the evidence item 1 as a tail node and the keyword 1 as the first edge. Such an evidence item representation entry serves as a part of the relevant information evidence item representation entry and is written into the evidence item knowledge graph of the target variant.

For the other parts of document 1, other documents in the document set, other evidence item representation entries of relevant information can be acquired in the similar manner.

The relevant information evidence item knowledge graph can be formed by traversing all relevant information evidence item representation entry acquired from the document set.

In practice, the method provided in the present application can be used to construct evidence item knowledge graphs for all or part of evidence items based on industry standards or guidelines.

When only some evidence items are judged by constructing an evidence item knowledge graph, other evidence items can be judged by other methods.

In practical implementation, according to Genetic Variant Classification Criteria and Guidelines, an evidence item standard knowledge entry may be extracted for the evidence items that are required to be judged based on at least one of patient and/or family records, classification result records and function verification records.

The extracted evidence items of the evidence item standard knowledge entry include: strong evidence of pathogenicity (PS2, PS3, and PS4), moderate evidence of pathogenicity (PM3, PM6), supporting evidence of pathogenicity (PP1, PP4, PP5), strong evidence of benign impact (BS3, BS4), and supporting evidence of benign impact (BP2, BP5, BP6).

Each of the above-mentioned evidence item standard knowledge entry is represented with a triad as a basic unit to form each of the evidence item standard knowledge representation entry.

All of the above evidence item standard knowledge representation entries are associated to form an evidence item standard knowledge graph.

For specific information, entities and/or relationships in the evidence item standard knowledge graph are identified and extracted from the selected target document set.

If the entities and relationships in a certain evidence item standard knowledge representation entry have matching contents, it can be derived that the contents recorded in the target document set meet the requirements of the evidence item; then, the respective entities and relationships in the evidence item standard knowledge representation entry are represented by the specific names recorded in the target document set, and transformed into the evidence item evidence representation entry.

The selected target document set is transversed, all the acquired evidence item evidence representation entries of the target variant are associated to form an evidence item knowledge graph corresponding to relevant information.

The method described in this embodiment can be used to accurately and efficiently acquire the information recorded in the document and judge whether the information meets the requirements of evidence items, and directly obtain the conclusion of evidence items of target variant in the selected target document set.

In some embodiments, the evidence items to be judged by at least one of functional inferences, computer predictions, and population records recited in the Standards and guidelines for the interpretation of sequence variants may also be judged by information sources selected from one or more biological information software predictions, public database records, or a combination thereof.

The function inference is the inference made on the influence on the function of the variant protein according to the known variant record of the target gene.

The target gene refers to the gene where the variant is located.

One or more known variants may exist and may be pathogenic variants and/or benign variants.

The known variant records include at least one of a sequence of the known variant, a location of the known variant, a segment where the known variant is located, a type of the known variant, and a proportion of each type of the known variant.

Computer prediction refers to the assessment of the impact of a variant on a gene or gene product by means of bioinformatics.

Effects include harmful effects and harmless effects.

Assessments include at least one of protein conservation assessment, nucleic acid conservation assessment and cleavage hazard assessment.

Population record refers to the presence of variant in a large population.

The presence includes allelic frequency, and may further include the number of homozygotes and/or hemizygotes.

In this embodiment, the evidence items judged based on at least one of the function inference, the computer prediction, and the population record may include: very strong evidence of pathogenicity (PVS1), strong evidence of pathogenicity (PS1), moderate evidence of pathogenicity (PM1, PM2, PM4, and PM5), supporting evidence of pathogenicity (PP2, PP3), stand-alone evidence of benign impact (BA1), strong evidence of benign impact (BS1, BS2), and supporting evidence of benign impact (BP1, BP3, BP4, and BP7).

In some embodiments, the functional inference evidence item may further include: very strong evidence of pathogenicity (PVS1), strong evidence of pathogenicity (PS1), moderate evidence of pathogenicity (PM1, PM4, and PM5), supporting evidence of pathogenicity (PP2), and supporting evidence of benign impact (BP1, BP3).

In some embodiments, the computer predictive evidence item may include: supporting evidence of pathogenicity (PP3) and supporting evidence of benign impact (BP4, BP7).

In some embodiments, population record evidence item may include moderate evidence of pathogenicity (PM2), stand-alone evidence of benign impact (BA1), and strong evidence of benign impact (BS1, BS2).

In some embodiments, the evidence items acquired in each of the above-mentioned embodiments may also be combined to achieve classification of the target variant according to combined standard rules for genetic variant classification established by the Standards and guidelines for the interpretation of sequence variants, and the classification result is selected from one of five categories, i.e., pathogenic, likely pathogenic, benign, likely benign, and variants of uncertain significance.

The construction of evidence item knowledge graph and its application in the variant interpretation are illustrated with specific variant as an example.

For example, in practical implementation, the content of evidence items of the target variant c. 417C>T of the TGFBI gene can be searched and judged in the target literature Romero P, Moraga M, Herrera L. Different phenotypes of lattice corneal dystrophy type I in patients with 417C>T (R124C) and 1762A>G (H572R) mutations in TGFBI (BIGH3)[J]. Molecular Vision, 2010, 16: 1601.

In the target document, the first relationship in the PS2 evidence item standard knowledge representation entry can be identified through a relationship keyword library matching, and thus the evidence sentence is determined to be "Paternity was confirmed, indicating the R124C mutation was a de novo mutation", where "de novo" is the first relationship in the PS2 evidence item standard knowledge representation entry.

With such an evidence sentence as the center, the variant entities, patient entities and second relationships in the PS2 evidence item standard knowledge representation entries are identified within the scope from 1 sentence before to after the evidence sentence.

In the evidence sentence, a pre-training model is used to identify the specific name "R124C" of the target variant.

In the latter sentence of the evidence sentence "Individual I-1 of Family Three does not carry that mutation", the patient entity "individual" is identified by keyword library matching.

In the evidence sentence, the second relation "paternity was confirmed" is identified by relationship keyword library matching.

Inference can be made according to the PS2 evidence item standard knowledge representation entry to learn that the contents recorded in this document are consistent with the PS2 evidence items, and PS2 evidence items can be given.

A variant entity in the PS2 evidence item standard knowledge representation entry is described using "R124C" or a standard name "TGFBI c.417C>T" of a target variant, a first relationship is described using "de novo"; a patient entity is described using "individual" and a second relationship is described using "paternity was confirmed"; and a PS2 evidence item standard knowledge representation entry is converted into a PS2 evidence item knowledge representation entry.

It will be appreciated that in this embodiment, the target document set contains only one selected document (i.e., target document), the evidence item knowledge graph for the target variant TGFBI c. 417C>T consists of only one PS2 evidence item knowledge representation entry.

In other embodiments, the target variant TGFBI c. 417C>T may also be searched in an established target document set, which includes over 60,000 documents.

The following document is supplemented: Romero P, Vogel M, Diaz J M, et al. Anticipation in familial lattice corneal dystrophy type I with R124C mutation in the TGFBI (BIGH3) gene[J]. Molecular Vision, 2008, 14: 829.

In this supplemented document, the first relationship in the PP1 evidence item standard knowledge representation entry can be identified through relationship keyword library matching, to determine that the evidence sentence is "The R124C mutation in TGFBI cosegregated with LCD type I in the investigated family", where "cosegregated" is the first relationship in the PP1 evidence item standard knowledge representation entry.

In the evidence sentence, a pre-training model is used to identify the specific name "R124C" of the target variant.

Inference can be made according to the PP1 evidence item standard knowledge representation entry to learn that the contents recorded in this document are consistent with the PP1 evidence items, and PP1 evidence items can be given.

A variant entity in the PP1 evidence item standard knowledge representation entry is described using "R124C" or a standard name "TGFBI c.417C>T" of a target variant; a first relationship is described using "cosegregated"; and a PP1 evidence item standard knowledge representation entry is converted into a PP1 evidence item knowledge representation entry.

It will be appreciated that in this embodiment, the output PP1 evidence item knowledge representation entry may be combined with the output PS2 evidence item knowledge representation entry in the previous case to form a more comprehensive evidence item knowledge graph of the target variant TGFBI c. 417C>T.

In addition, in the established document set, if no matching content is identified for the exemplified PS4 evidence item, there is no output for the PS4 evidence item knowledge representation entry, and there is no PS4 evidence item knowledge representation entry in the evidence item knowledge graph of target variant TGFBI c. 417C>T.

In other embodiments, 22,000 genetic tumor-related documents from public databases maybe identified as the target document set and manually interpreted and reviewed by an interpretation expert.

By comparing the judgment results of the supporting evidence of pathogenicity PP1, strong evidence of pathogenicity PS4 and strong evidence of pathogenicity PS2 acquired using the method of the present application with the above manual interpretation results, the F1 values of the respective evidence items are 89.36%, 62.07% and 80%, respectively.

For other evidence items of the Standards and guidelines for the interpretation of sequence variants, and evidence items based on other industry standards or guidelines, the same method as the present application can be adopted to extract the evidence item standard entry, construct the evidence item standard knowledge graph, and construct the evidence item knowledge graph, to realize the judgment of evidence items.

During the research and development process, the inventors found that in the practical operation of variant interpretation, the comprehensive information of target variant shall be collected, it shall be sequentially judge whether each information meets the requirements of each evidence item, and then the classification of target variant shall be determined based on all the evidence items. Nearly half of 28 evidence items are judged depending on the information recorded in the documents. That is to say, firstly, it is necessary to retrieve the documents in which the target variant is recorded from a mass of published documents; secondly, it is necessary to further extract the specific content of the recorded target variant from the retrieved documents; then it is judged whether one or at least one of the 28 evidence items is met according to the specific content recorded; and finally, one or more evidence items for the target variant are determined.

In the related art, the determination of evidence items is mainly performed by means of manual search and reading judgment one by one. This method is not feasible to a large number of updating documents. Moreover, it is a tedious process to determine whether the information meets the requirements of evidence items; the manual judgment is not only prone to omission of information, but also depends on personal professional knowledge reserves. Different people may have different understandings of the same content, which will lead to different results. In addition, when the same documents are retrieved by different personnel at different times, the retrieval, reading and judgment need to be repeated manually, which is inefficient.

However, in the present application, the judgment content of at least one evidence item is extracted based on the target principle, the acquired at least one evidence item standard knowledge entry is represented to construct an evidence item standard knowledge graph, and the information recorded in the target document set is inferred based on the evidence item standard knowledge graph, to acquire the evidence item knowledge graph. In this way, the evidence item knowledge graph can be correspondingly updated based on the change of the document, and thus the method of the present application is applicable to mass and rapidly updating documents.

In addition, in the present application, by determining the evidence item of the target variant through the constructed evidence item knowledge graph, the information recorded in the document can be acquired comprehensively, quickly, accurately and efficiently without subjective judgment of the user, and it can be judged whether the information meets the requirements of the evidence item, thereby avoiding the influence of the subjective judgment on the judgment result and effectively avoiding the information omission caused by the manual method. At the same time, it has lower requirements for the judgment, allowing the operations of determining the evidence items to be simpler and more convenient, and significantly improving the accuracy of the judgment results.

According to the variant evidence item determination method provided in the embodiment of the present application, the judgment content of at least one evidence item is extracted based on the target principle; the at least one evidence item standard knowledge entry acquired is represented to construct the evidence item standard knowledge graph; and the information recorded in the target document set is inferred based on the evidence item standard knowledge graph, to acquire the evidence item knowledge graph. In the subsequent application process, the required target evidence item corresponding to the target variant can be quickly and accurately acquired from massive literature, having a broad application perspective. In addition, the repeat manual search can be omitted, which saves manpower and time cost and improves the efficiency of determining evidence items. Besides, the influence of user's subjective judgment on final result can be effectively avoided, thereby improving the uniformity of the judgment result and having higher judgment accuracy and precision.

The embodiments of the present application provide a variant evidence item determination method, and an execution subject thereof can be a variant evidence item determination apparatus. In the embodiments of the present application, a variant evidence item determination method provided in the embodiments of the present application is described by taking an example in which the variant evidence item determination apparatus performs a variant evidence item determination method.

The embodiments of the present application further provide a variant evidence item determination apparatus.

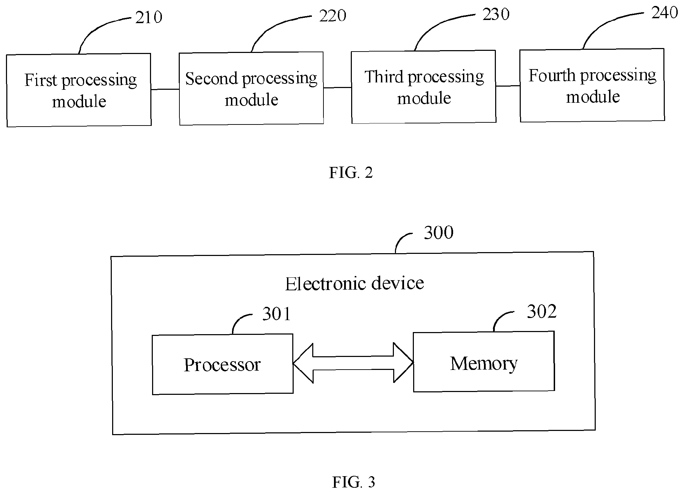

As illustrated in FIG. 2, the variant evidence item determination apparatus includes a first processing module 210, a second processing module 220, a third processing module 230 and a fourth processing module 240.

The first processing module 210 is configured to extract judgment content of at least one evidence item based on a target principle, to acquire at least one evidence item standard knowledge entry.

The second processing module 220 is configured to represent the evidence item standard knowledge entry to acquire an evidence item standard knowledge graph.

The third processing module 230 is configured to: identify information recorded in a target document set, and match the information with the evidence item standard knowledge graph, to acquire an evidence item knowledge graph.

The fourth processing module 240 is configured to determine, based on the evidence item knowledge graph, a target evidence item corresponding to a target variant.

According to the variant evidence item determination apparatus provided in the embodiment of the present application, the judgment content of at least one evidence item is extracted based on the target principle; the at least one evidence item standard knowledge entry acquired is represented to construct the evidence item standard knowledge graph; and the information recorded in the target document set is inferred based on the evidence item standard knowledge graph, to acquire the evidence item knowledge graph. In the subsequent application process, the required target evidence item corresponding to the target variant can be quickly and accurately acquired from massive literature, having a broad application perspective. In addition, the repeat manual search can be omitted, which saves manpower and time cost and improves the efficiency of determining evidence items. Besides, the influence of user's subjective judgment on final result can be effectively avoided, thereby improving the uniformity of the judgment result and having higher judgment accuracy and precision.

In some embodiments, the second processing module 220 may be further configured to: the evidence item standard knowledge entry with a triad as a basic unit, to acquire at least one evidence item standard knowledge representation entry; and combine the at least one evidence item standard knowledge representation entry, to acquire the evidence item standard knowledge graph.

In some embodiments, the third processing module 230 may be further configured to: acquire at least part of the entity and/or relationship included by the evidence item standard knowledge representation entry within a first target range; and in a case that the information recorded in the target document set matches the at least part of the entity and/or relationship, represent the evidence item standard knowledge representation entry, and construct the evidence item knowledge graph.

In some embodiments, the third processing module 230 may be further configured to: determine, based on the target document set, a sentence containing an entity and/or relationship of a first triad in an evidence item standard knowledge representation entry as an evidence sentence; and in a case that other entities and/or relationships in the evidence item standard knowledge representation entry are acquired by searching a context of the evidence sentence, transform, based on specific names corresponding to the other entities and/or relationships recorded in the target document set, the evidence item standard knowledge representation entry corresponding to the other entities and/or relationships, to generate the evidence item knowledge graph.

In some embodiments, the third processing module 230 may be further configured to: determine an evidence sentence based on a type of the evidence item standard knowledge representation entry; and generate the evidence item knowledge graph based on the evidence sentence and information recorded in the target document set.

In some embodiments, the first processing module 210 may be further configured to: extract, based on the target principle, the judgment content of the at least one evidence item based on entities and relationships of the entities, to acquire the at least one evidence item standard knowledge entry. The entities comprise at least one of a variant entity, a disease entity, a patient entity, an index entity, a functional entity, or an evidence item.

The variant evidence item determination apparatus in the embodiments of the present application may be an electronic device or may be a component in an electronic device, such as an integrated circuit or a chip. The electronic device may be a terminal or may be a device other than a terminal. Illustratively, the electronic device may be, for example, a mobile phone, a tablet computer, a notebook computer, a palm computer, a vehicle-mounted electronic device, a Mobile Internet Device (MID), an Augmented Reality (AR)/Virtual Reality (VR) Device, a robot, a wearable device, an Ultra-mobile Personal Computer (UMPC), a netbook or a Personal Digital Assistant (PDA), etc., and may also be a server, a Network Attached Storage (NAS), a personal computer (PC), a television (TV), an assailant, a self-service machine, etc., which is not specifically limited herein.

The variant evidence item determination apparatus in the embodiment of the present application may be an apparatus having an operating system. The operating system may be an Android operating system, may be an IOS operating system, and may also be other possible operating systems, which is not limited by the embodiments of the present application.

The variant evidence item determination apparatus provided in the embodiments of the present application can implement various processes implemented in the embodiment of the variant evidence item determination method of FIG. 1, which is not repeated herein.

In some embodiments, as illustrated in FIG. 3, the embodiments of the present application further provide an electronic device 300. The electronic device 300 includes a processor 301, a memory 302, and a computer program stored on the memory 302 and executable on the processor 301. The computer program, when executed by the processor 301, implements the various processes of the above-mentioned embodiment of the variant evidence item determination method, and thus it can achieve the same technical effects, which are not repeated herein.

It should be noted that the electronic devices in the embodiments of the present application include mobile electronic devices and non-mobile electronic devices described above.

The embodiments of the present application further provide a non-transitory computer-readable storage medium on which a computer program is stored. When the computer program is executed by a processor, various processes of the above-mentioned embodiments of the variant evidence item determination method are implemented, and the same technical effect can be achieved, which are not repeated herein.

The processor is a processor in the electronic device described in the above embodiments. The readable storage medium includes a computer readable storage medium such as a computer read only memory (ROM), a random-access memory (RAM), a magnetic or optical disk, etc.

The embodiments of the present application further provide a computer program product including a computer program. The computer program, when executed by a processor, implements the variant evidence item determination.

The processor is a processor in the electronic device described in the above embodiments. The readable storage medium includes a computer readable storage medium such as a computer read only memory (ROM), a random access memory (RAM), a magnetic or optical disk, etc.

The embodiments of the present application further provide a chip including a processor and a communication interface. The communication interface is coupled with the processor. The processor is configured to run a program or instructions to implement the various processes of the above-mentioned embodiments of the variant evidence item determination method. Thus, the chip can achieve the same technical effect, and in order to avoid repetition, which are not repeated herein.

It should be understood that chip mentioned in embodiments of the present application may also be referred to as system-on-chip, system-on-a-chip, SoC, System on a Chip, etc.

It should be noted that, in this document, the terms "includes," "including," or any other variant thereof, are intended to cover a non-exclusive inclusion, such that a process, method, article, or apparatus including a list of elements does not include only those elements but may include other elements not expressly listed or inherent to such process, method, article, or apparatus. An element proceeded by the phrase "includes a....." does not, without more constraints, preclude the existence of additional identical elements in the process, method, article, or apparatus that includes the element. Further, it should be noted that the scope of the methods and apparatus in the embodiments of the present application is not limited to performing the functions in the order illustrated or discussed, but may include performing the functions in a substantially simultaneous manner or in a reverse order based on the functions recited, e.g., the methods described may be performed in an order different than that described, and various steps may be added, omitted, or combined. Additionally, features described with reference to certain examples may be combined in other examples.

Through the description of the foregoing embodiments, a person skilled in the art would know that the method of the foregoing embodiments may be implemented by software plus a necessary general hardware platform, and certainly may also be implemented by hardware, but in many cases, the former is a better implementation. Based on such understanding, the technical solutions of the present application or portions thereof contributing to the prior art may be embodied in the form of a computer software product, which is stored in a storage medium (such as ROM/RAM, magnetic disk, optical disk) and includes instructions for enabling a terminal (which may be a mobile phone, a computer, a server, or a network device) to execute the methods according to the embodiments of the present application.

While the present application has been described with reference to the particular illustrative embodiments, it can be understood that the application is not limited to the disclosed embodiments, but is intended to cover various modifications, equivalent arrangements, and equivalents thereof, which may be made by a person skilled in the art without departing from the spirit and scope of the application as defined by the appended claims.

In the description of the present specification, reference to the description of "one embodiment," "some embodiments," "a schematic embodiment," "an example," "a specific example," or "some examples" etc. means that a particular feature, structure, material, or characteristic described in connection with the embodiment or example is included in at least one embodiment or example of the present application. In this specification, schematic representations of the above terms do not necessarily refer to the same embodiment or example. Furthermore, the particular features, structures, materials, or characteristics described may be combined in any suitable manner in one or at least one embodiment or example.

While embodiments of the present application have been illustrated and described, it will be understood by a person skilled in the art that: various changes, modifications, substitutions and alterations can be made to the embodiments without departing from the principles and spirit of the application, the scope of which is defined by the claims and their equivalents.

## Claims

1. A variant evidence item determination method, comprising:
extracting judgment content of at least one evidence item based on a target principle, to acquire at least one evidence item standard knowledge entry;
representing the evidence item standard knowledge entry to acquire an evidence item standard knowledge graph;
identifying information recorded in a target document set, and matching the information with the evidence item standard knowledge graph, to acquire an evidence item knowledge graph; and
determining, based on the evidence item knowledge graph, a target evidence item corresponding to a target variant.

2. The variant evidence item determination method according to claim 1, wherein said representing the evidence item standard knowledge entry to acquire the evidence item standard knowledge graph comprises:
representing the evidence item standard knowledge entry with a triad as a basic unit, to acquire at least one evidence item standard knowledge representation entry; and
combining the at least one evidence item standard knowledge representation entry, to acquire the evidence item standard knowledge graph.

3. The variant evidence item determination method according to claim 1, wherein the evidence item standard knowledge representation entry takes a variant entity as a head node and an evidence item as a tail node.

4. The variant evidence item determination method according to any one of claims 1 to 3, wherein in the evidence item knowledge graph, respective nodes other than the tail node and edges are each represented by a specific name recorded in the target document set.

5. The variant evidence item determination method according to any one of claims 1 to 3, wherein said identifying information recorded in the target document set, and matching the information with the evidence item standard knowledge graph, to acquire the evidence item knowledge graph comprises:
acquiring at least part of an entity and/or relationship comprised in an evidence item standard knowledge representation entry within a first target range; and
in a case that the information recorded in the target document set matches the at least part of the entity and/or relationship, representing the evidence item standard knowledge representation entry to construct the evidence item knowledge graph.

6. The variant evidence item determination method according to any one of claims 1 to 3, wherein said identifying information recorded in the target document set, and matching the information with the evidence item standard knowledge graph, to acquire the evidence item knowledge graph comprises:
determining, based on the target document set, a sentence containing an entity and/or relationship of a first triad in an evidence item standard knowledge representation entry as an evidence sentence; and
in a case that other entities and/or relationships in the evidence item standard knowledge representation entry are acquired by searching a context of the evidence sentence, transforming, based on specific names corresponding to the other entities and/or relationships recorded in the target document set, the evidence item standard knowledge representation entry corresponding to the other entities and/or relationships, to generate the evidence item knowledge graph.

7. The variant evidence item determination method according to any one of claims 1 to 3, wherein said identifying information recorded in the target document set, and matching the information with the evidence item standard knowledge graph, to acquire the evidence item knowledge graph comprises:
determining an evidence sentence based on a type of the evidence item standard knowledge representation entry; and
generating the evidence item knowledge graph based on the evidence sentence and the information recorded in the target document set.

8. The variant evidence item determination method according to any one of claims 1 to 3, wherein said extracting, based on the target principle, the judgment content of the at least one evidence item, to acquire the at least one evidence item standard knowledge entry comprises:
extracting, based on the target principle, the judgment content of the at least one evidence item based on entities and relationships of the entities, to acquire the at least one evidence item standard knowledge entry,
wherein the entities comprise at least one of the following: a variant entity, a disease entity, a patient entity, an index entity, a functional entity, or an evidence item.

9. A variant evidence item determination apparatus, comprising:
a first processing module configured to extract judgment content of at least one evidence item based on a target principle, to acquire at least one evidence item standard knowledge entry;
a second processing module configured to represent the evidence item standard knowledge entry to acquire an evidence item standard knowledge graph, wherein the number of items comprised in the evidence item standard knowledge graph matches the evidence item standard knowledge entry;
a third processing module configured to: identify information recorded in a target document set, and match the information with the evidence item standard knowledge graph, to acquire an evidence item knowledge graph; and
a fourth processing module configured to determine, based on the evidence item knowledge graph, a target evidence item corresponding to a target variant.

10. An electronic device, comprising:
a memory;
a processor; and
a computer program stored on the memory and executable on the processor,
wherein the processor, when executing the program, implements the variant evidence item determination method according to any one of claims 1 to 8.
